(19) Europäisches Patentamt

European Patent Office

Office européen des brevets

(11) Veröffentlichungsnummer: **0 317 039 B1**

(12) **EUROPÄISCHE PATENTSCHRIFT**

(45) Veröffentlichungstag der Patentschrift: **30.09.92**

(21) Anmeldenummer: **88202644.6**

(22) Anmeldetag: **09.11.88**

(51) Int. Cl.5: **A61B 3/10**

(54) **Spaltlampengerät mit Umfeldbeleuchtung.**

(30) Priorität: **10.11.87 DE 8714962 U**

(43) Veröffentlichungstag der Anmeldung:
**24.05.89 Patentblatt 89/21**

(45) Bekanntmachung des Hinweises auf die
Patenterteilung:
**30.09.92 Patentblatt 92/40**

(84) Benannte Vertragsstaaten:
**ES**

(56) Entgegenhaltungen:
**WO-A-84/01110**
**WO-A-85/00966**
**US-A- 4 102 565**

(73) Patentinhaber: **Firma Carl Zeiss**
**Carl-Zeiss-Strasse 4-54**
**W-7082 Oberkochen(DE)**

(72) Erfinder: **Müller, Ortwin**
**Kolpingstr.34**
**W-7080 Aalen(DE)**
Erfinder: **Geiss, Günter**
**Eschenweg 14**
**W-7923 Königsbronn(DE)**
Erfinder: **Stopar, Victor**
**Mozartweg 13**
**W-7082 Oberkochen(DE)**

EP 0 317 039 B1

**Beschreibung**

Die Erfindung betrifft ein Spaltlampengerät, bestehend aus einem Stereomikroskop, einer um eine senkrechte Achse drehbaren Spaltbeleuchtungseinrichtung, einer Umfeldbeleuchtungseinrichtung und einer das Stereomikroskop und die Beleuchtungseinrichtungen koppelnde Gerätemechanik. Ein derartiges Spaltlampengerät ist Beispielsweise aus WO-A-84/01110 bekannt.

Spaltlampengeräte, die zusätzlich zur Spaltbeleuchtungseinrichtung eine Umfeldbeleuchtungseinrichtung aufweisen, sind als sogenannte Photospaltlampen bekannt und beispielsweise in der Schrift "Augenuntersuchung mit der Spaltlampe" herausgegeben von Carl Zeiss, Oberkochen beschrieben. Nachteilig bei diesen bekannten Photospaltlampen ist, daß der Einfallswinkel der Umfeldbeleuchtungseinrichtung nur geändert werden kann, wenn diese Beleuchtungseinrichtung zusammen mit dem Steromikroskop um die Drehachse der Gerätemechanik geschwenkt wird, um die auch die Spaltbeleuchtungseinrichtung schwenkbar ist.

Die Ebene des Spaltbildes und die Mikroskop-Schärfenebene enthalten die virtuelle Verlängerungslinie dieser Drehachse. Mittels einer Instrumentenbasis läßt sich diese Ebene in X, Y, Z-Richtung an den Ort der Untersuchung im Auge verschieben. Die mechanische Lagerung dieser gemeinsamen Drehachse für die Spaltbeleuchtungseinrichtung und für das Stereomikroskop kann je nach Ausführungsart des Spaltlampengerätes oberhalb oder unterhalb des Kopfes des Patienten angeordnet sein.

Der vorliegenden Erfindung liegt die Aufgabe zugrunde, bekannte Spaltlampengeräte mit einer zusätzlichen Beleuchtungseinrichtung auszustatten, deren Einstrahlrichtung unabhängig von anderen Geräteteilen und von der Spaltbeleuchtungseinrichtung geändert werden kann.

Diese Aufgabe wird entsprechend den Merkmalen des kennzeichnenden Teil des Patentanspruches 1 gelöst.

Vorteilhafte Ausführungsformen der Erfindung zeichnen sich durch die im kennzeichnenden Teil der Ansprüche 2 bis 4 wiedergegebenen Merkmale aus.

Die mit der Erfindung erzielten Vorteile bestehen insbesondere darin, daß die Achsen der Spaltbeleuchtungseinrichtung und der Umfeldbeleuchtungseinrichtung isozentrisch zueinander angeordnet werden können, womit gewährleistet ist, daß sich der Leuchtfleck der Zusatzbeleuchtung stets in der Fokusebene der Spaltbeleuchtungseinrichtung und des Stereomikroskops befindet. Ein weiterer Vorteil der Erfindung besteht darin, daß anstelle der Umfeldbeleuchtung eine Laserlichtquelle angeordnet werden kann. Mit einer derartigen Laserspaltlampe ist es möglich, die Einstrahlrichtung des Lasers unabhängig von der Einblickrichtung des Mikroskops und der Einstrahlrichtung der Spaltbeleuchtung zu wählen.

Ausführungsbeispiele der Erfindung sind in der Zeichnung dargestellt und werden im folgenden näher beschrieben. Es zeigen

Figur 1    eine perspektivische Darstellung eines Spaltlampengerätes mit einer zusätzlichen, um eine senkrechte Achse drehbaren Umfeldbeleuchtung;

Figur 2    eine Schnittdarstellung des in Figur 1 gezeigten Spaltlampengerätes;

Figur 3    eine Schnittdarstellung einer Laserspaltlampe;

Figur 4    eine Schnittdarstellung eines Spaltlampengerätes, bei der die Umfeldbeleuchtung oberhalb des Patientenauges angeordnet ist.

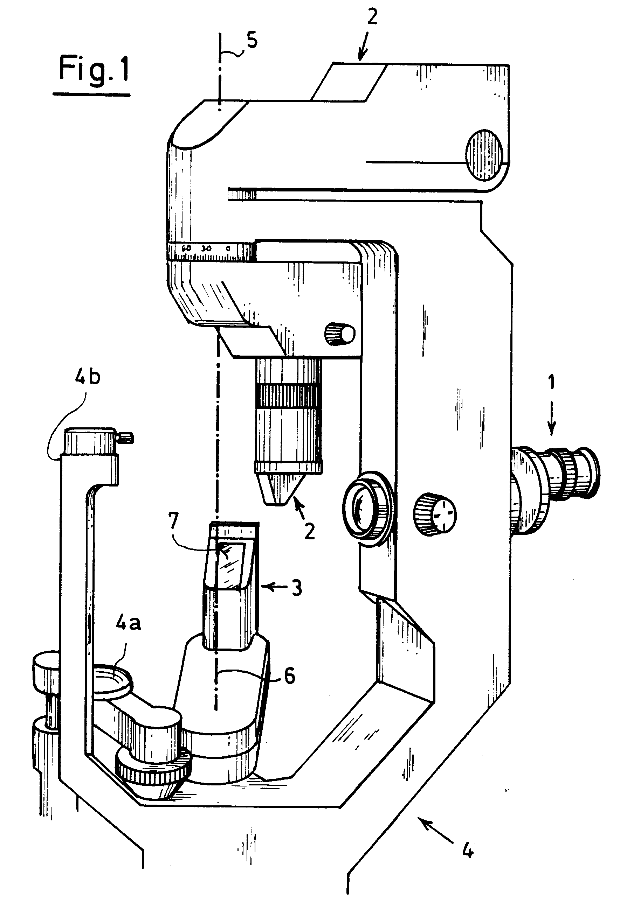

In der Darstellung der Figur 1 ist mit dem Bezugszeichen (1) ein Stereomikroskop bezeichnet, mit (2) eine Spaltbeleuchtungseinrichtung, mit (3) eine Umfeldbeleuchtungseinrichtung und mit (4) die Gerätemechanik. Mit (4a) ist die Kinnstütze, mit (4b) die Vorrichtung zur Halterung der Kopfstütze für den Patienten gekennzeichnet. Die Beleuchtungseinrichtung (3) ist um die Achse (6) drehbar, die isozentrisch zur Drehachse (5) verläuft. Die Beleuchtungseinrichtung (3) ist mit einem Fenster (7) für den Durchlaß der Beleuchtungsstrahlen abgeschlossen.

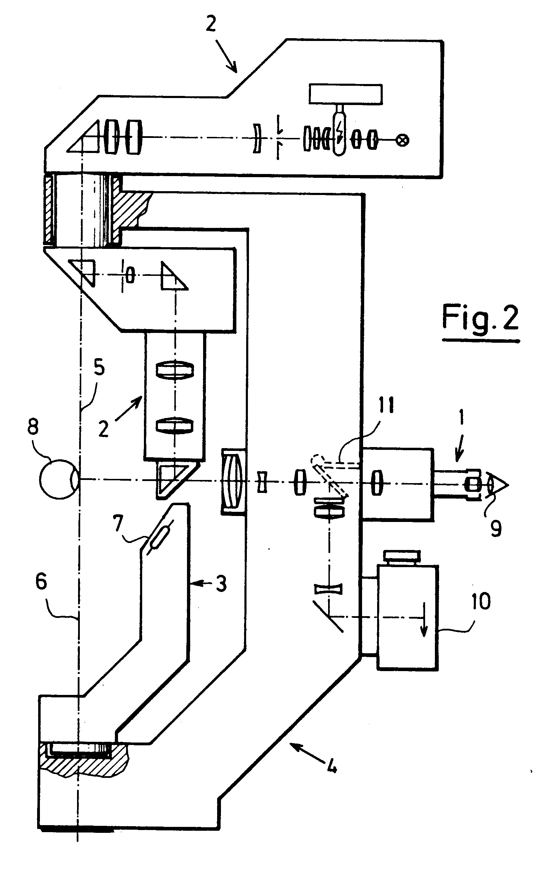

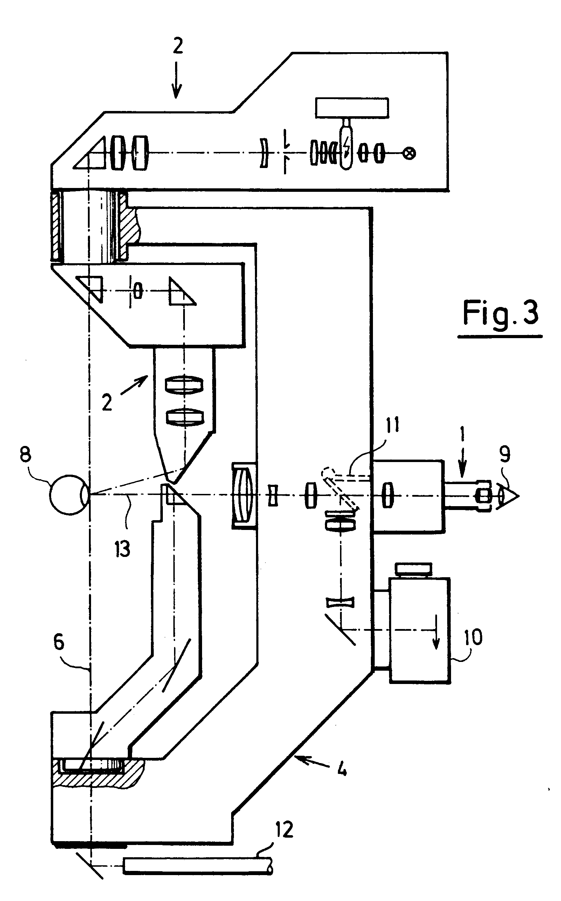

In der Schnittdarstellung der Figur 2 sind die optischen Elemente für das Spaltlampengerät eingetragen. Sie sind im einzelnen nicht bezeichnet, weil sie sich aus dem vorbekannten Stand der Technik ergeben. Das Patientenauge ist in dieser Darstellung mit dem Bezugszeichen (8) gekennzeichnet, das Beobachterauge mit (9). Das Bezugszeichen (10) kennzeichnet eine photographische Einrichtung für die Dokumentation, auf die im Bedarfsfall über den Klappspiegel (11) der Beobachtungsstrahl gelenkt werden kann. Im übrigen sind für gleiche Geräteteile die gleichen Bezugszeichen wie in der Figur 1 verwendet. Die Darstellung der Figur 3 zeigt bis auf eine Laserlichtquelle (12) die gleiche Anordnung wie in Figur 2. Die Strahlung der Laserlichtquelle (12) kann in dieser Vorrichtung anstelle einer Umfeldbeleuchtung für Therapiezwecke auf das Patientenauge (8) gerichtet werden.

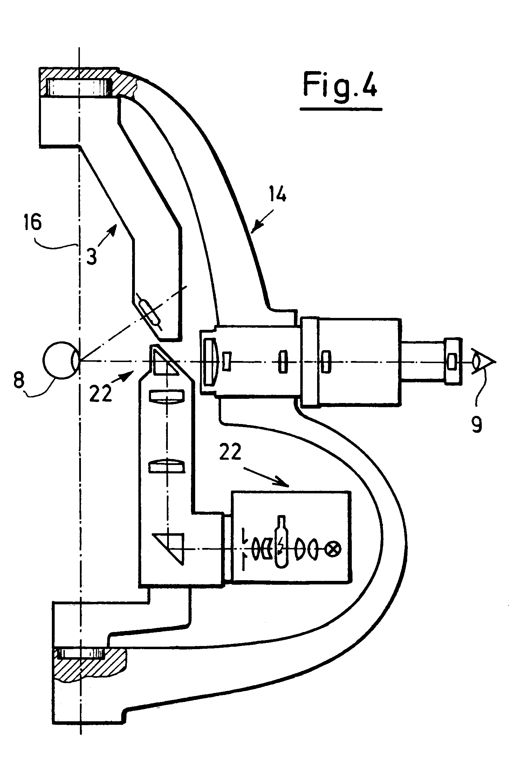

In der Darstellung der Figur 4 ist ein Spaltlampengerät mit einer Gerätemechanik (14) gezeigt, bei dem die Spaltbeleuchtungseinrichtung (22) unterhalb des Patientenauges (8) und die Umfeldbeleuchtungseinrichtung (3) oberhalb des Patientenauges (8) angeordnet ist. Die Drehachse (16) der Umfeldbeleuchtungseinrichtung verläuft isozentrisch zur Drehachse der Spaltbeleuchtungseinrichtung.

**Patentansprüche**

1.  Spaltlampengerät, bestehend aus einem Stereomikroskop (1), einer um eine senkrechte Achse (5) drehbaren Spaltbeleuchtungseinrichtung (2), einer Umfeldbeleuchtungseinrichtung (3) und einer das Stereomikroskop (1) und die Beleuchtungseinrichtungen (2,3) koppelnde Gerätemechanik (4), dadurch gekennzeichnet, daß die Umfeldbeleuchtungseinrichtung (3) unabhängig von anderen Geräteteilen um eine senkrechte Achse (6) drehbar ist.

2.  Spaltlampengerät nach Anspruch 1, dadurch gekennzeichnet, daß die Drehachse (6) der Umfeldbeleuchtungseinrichtung (3) isozentrisch zur Drehachse (5) der Spaltbeleuchtungseinrichtung (2) ist.

3.  Spaltlampengerät nach Anspruch 2, dadurch gekennzeichnet, daß die Umfeldbeleuchtungseinrichtung (3) unterhalb des Patientenkopfes mit der Gerätemechanik (4) verbunden ist.

4.  Spaltlampengerät nach Anspruch 2, dadurch gekennzeichnet, daß die Umfeldbeleuchtung (3) oberhalb des Patientenkopfes mit der Gerätemechanik (4) verbunden ist.

**Claims**

1.  Slit lamp apparatus comprising a stereomicroscope (1), a slit illuminating device (2) rotatable about a vertical axis (5), a peripheral illuminating device (3) and an apparatus mechanism (4) coupling the stereomicroscope (1) and the illuminating devices (2, 3), characterized in that the peripheral illuminating device (3) is rotatable about a vertical axis (6) independently of other apparatus parts.

2.  Slit lamp apparatus of claim 1, characterized in that the rotational axis (6) of the peripheral illuminating device (3) is isocentric to the rotational axis (5) of the slit illuminating device (2).

3.  Slit lamp apparatus of claim 2, characterized in that the peripheral illuminating device (3) is connected to the apparatus mechanism (4) below the head of the patient.

4.  Slit lamp apparatus of claim 2, characterized in that the peripheral illumination (3) is connected to the apparatus mechanism (4) above the head of the patient.

**Revendications**

1.  Appareil à lampe à fente, constitué d'un stéréomicroscope (1), d'un dispositif d'éclairage de fente (2) rotatif autour d'un axe vertical (5), d'un dispositif d'éclairage du champ périphérique (3) et d'un mécanisme d'appareil (4) accouplant le stéréomicroscope (1) et les dispositifs d'éclairage (2, 3), caractérisé en ce que le dispositif d'éclairage du champ périphérique (3) est rotatif autour d'un axe vertical (6) indépendamment d'autres parties de l'appareil.

2.  Appareil à lampe à fente selon la revendication 1, caractérisé en ce que l'axe de rotation (6) du dispositif d'éclairage du champ périphérique (3) possède le même centre que l'axe de rotation (5) du dispositif d'éclairage de fente (2).

3.  Appareil à lampe à fente selon la revendication 2, caractérisé en ce que le dispositif d'éclairage du champ périphérique (3) est relié au mécanisme d'appareil (4) au-dessous de la tête du patient.

4.  Appareil à lampe à fente selon la revendication 2, caractérisé en ce que le dispositif d'éclairage du champ périphérique (3) est relié au mécanisme d'appareil (4) au-dessus de la tête du patient.

# Fig.1

Fig.2

EP 0 317 039 B1

Fig.3

6

Fig.4